# EUROPÄISCHE PATENTSCHRIFT

(11) **EP 2 188 247 B1**
(45) Veröffentlichungstag und Bekanntmachung des Hinweises auf die Patenterteilung: **02.05.2018**
(21) Anmeldenummer: 08787181.0
(22) Anmeldetag: 13.08.2008
(51) Int. Cl.: B01J 12/00, C07C 263/10

(54) **VERFAHREN ZUR HERSTELLUNG VON ISOCYANATEN**
METHOD FOR PRODUCING ISOCYANATES
PROCÉDÉ DE PRODUCTION D'ISOCYANATES

(30) Priorität: 30.08.2007 EP 07115316
(43) Veröffentlichungstag der Anmeldung: 26.05.2010
(73) Patentinhaber: BASF SE, 67056 Ludwigshafen am Rhein (DE)
(72) Erfinder: OLBERT, Gerhard, 69221 Dossenheim (DE); MATTKE, Torsten, 67251 Freinsheim (DE); KNÖSCHE, Carsten, 67150 Niederkirchen (DE); DAISS, Andreas, 67146 Deidesheim (DE); DENECKE, Jens, 67063 Ludwigshafen (DE)
(74) Vertreter: BASF IP Association
(86) Internationale Anmeldenummer: PCT/EP2008/060630
(87) Internationale Veröffentlichungsnummer: WO 2009/027232

(56) Entgegenhaltungen:
- EP-A- 1 275 639
- EP-A- 1 362 847
- EP-A- 1 526 129
- WO-A1-2008/055898
- DE-A1-102005 042 392

## Beschreibung

Die vorliegende Erfindung betrifft ein Verfahren zur Herstellung von Isocyanaten.

Zur Herstellung von Isocyanaten durch Phosgenierung der korrespondierenden Amine besteht prinzipiell die Möglichkeit einer Flüssigphasen- oder einer Gasphasenphosgenierung. Die Gasphasenphosgenierung zeichnet sich dadurch aus, daß die Reaktionsbedingungen so gewählt werden, daß zumindest die Reaktionskomponenten Diamin, Diisocyanat und Phosgen, bevorzugt jedoch sämtliche Edukte, Produkte und Reaktionszwischenprodukte, bei diesen Bedingungen, besonders bevorzugt bis zum Abschluß der Reaktion im Wesentlichen gasförmig sind. Die vorliegende Erfindung betrifft ausschließlich die Gasphasenphosgenierung. "Im Wesentlichen" bedeutet dabei zu mindestens 50 Gew% bezogen auf sämtlich Reaktionskomponenten, bevorzugt zu mindestens 66%, besonders bevorzugt zu mindestens 75%, ganz besonders bevorzugt zu mindestens 85%, insbesondere zu mindestens 90% und speziell zu mindestens 95%. Denkbar wäre die zwischenzeitliche Bildung von flüssigen Tröpfchen und/oder zwischenzeitlich gebildeten partikulären Feststoffen, die in einer gasförmigen Umgebung reagieren.

EP 1 275 639 A1 beschreibt die Gasphasenphosgenierung von (cyclo)aliphatischen Diaminen in einer Reaktionszone mit Einengungen der Wände.

In der Mischeinrichtung werden die amin- und phosgenhaltigen Eduktströme koaxial einer Mischzone zugeführt, wobei der phosgenhaltige Eduktstrom innen und der aminhaltige Eduktstrom außen geführt wird. Im Bereich der Zusammenführung der Eduktströme also der Reaktionszone erfolgt eine weitere Reduzierung oder leichte Vergrößerung des Strömungsquerschnittes, so daß aufgrund der Volumenzunahme im Laufe der Reaktion durch Expansion des Gases infolge der Exothermie der Reaktion die Strömungsgeschwindigkeit ansteigt.
Nachteilig bei dieser Anordnung ist, dass der Aminstrom koaxial außen geführt wird. Dadurch kann es zur Feststoffbildung an den Wänden der Mischeinrichtung kommen, da an den Wänden das Amin gegenüber dem Phosgen im Überschuss vorliegt, was die Nebenproduktbildung begünstigt.

Ebenfalls in EP 1275639A1 wird beschrieben, dass in der Mischvorrichtung vor der Zusammenführung der Eduktströme eine Verdrallung der Eduktströme erfolgen soll, so dass die turbulenten Schwankungsgeschwindigkeiten in den Eduktströmen erhöht sind und die Vermischung bei der Zusammenführung der beiden Eduktströme dann rascher erfolgt.

EP 1526129 A1 beschreibt die Erhöhung einer Turbulenz in einer Vermischungdüse durch drallerzeugende Einbauten. Dadurch wird eine tangentiale Verwirbelung des gesamten Stromes erzeugt, der sich jedoch nicht signifikant auf die Vermischung der unterschiedlichen Ströme miteinander auswirkt.

EP 1 275 640 A1 beschreibt die Gasphasenphosgenierung von (cyclo)aliphatischen Di- und Triaminen in einem Mischrohr mit Reaktor, in dem die Gasströmung im Mischbereich beschleunigt wird.

Nachteilig bei diesem Verfahren ist, dass nicht sofort zu Beginn der Vermischung die maximale Geschwindigkeitsdifferenz zwischen den Reaktandenströmen erreicht wird und damit auch nicht die minimal mögliche Mischzeit erzielt wird.

DE 10359627 A1 offenbart eine Gasphasenphosgenierung, in der Amin durch einen konzentrischen Ringspalt zwischen zwei Phosgenströme eingemischt wird, wobei die Flächen, durch die die Phosgenströme fließen, in einem Verhältnis von 1:0,5 bis 1:4 stehen.

Aus der internationalen Anmeldung WO 2007/028715 ist ein Verfahren bekannt, in dem Amin und Phosgen über Ringspalte, also ringförmig geschlossene Spalte dosiert werden.

In allen diesen Schriften werden ausschließlich glatte Düsen offenbart, turbulenzerzeugende Einbauten werden höchstens über Verdrallungen offenbart.

Keine der im genannten Stand der Technik bekannten Mischvorrichtungen hat es bisher bewirkt, die Bildung von Feststoffen an der Zusammenführung des Amin- und des Phosgenstromes dauerhaft zufriedenstellend zu unterdrücken.

Aufgabe der vorliegenden Erfindung war es nun, eine Reaktionsführung für eine Gasphasenphosgenierung zu entwickeln, mit der eine großtechnische Durchführung möglich wird und die unanfällig gegenüber Verstopfungen wird, in dem lediglich eine geringe Ablagerungsneigung von Feststoff besteht.

Die Aufgabe wird gelöst durch Verfahren zur Herstellung von Isocyanaten durch Umsetzung der korrespondierenden Amine mit Phosgen in Gegenwart von mindestens einem, bevorzugt genau einem Inertmedium, in der Gasphase, durch Inkontaktbringen fluider Ströme von Amin, Phosgen sowie Inertmedium in mindestens einer Mischeinrichtung und anschließende Reaktion von Amin und Phosgen miteinander, in dem man in der Mischeinrichtung zumindest zwischen jeden vorhandenen Aminstrom und jeden vorhandenen Phosgenstrom ein Inertmedium dosiert.

Ein weiterer Gegenstand der vorliegenden Erfindung ist die Verwendung von Dreistoffmischdüsen zur Dosierung fluider von Inertmedium zwischen fluide Ströme von jeden vorhandenen Aminstrom und jeden vorhandenen Phosgenstrom in der Gasphasenphosgenierung.

Ein weiterer Gegenstand der vorliegenden Erfindung ist die Verwendung von Dreistoffmischdüsen zur Dosierung fluider Ströme von Amin, Phosgen und Inertmedium in der Flüssigphasenphosgenierung.

Dieses erfindungsgemäße Prinzip kann generell auf Vorgänge angewendet werden, in denen eine schnelle Mischung fluider, also gasförmiger oder flüssiger, Stoffe gewünscht wird, insbesondere bei chemischen Reaktionen.

Derartige chemische Reaktionen sind bevorzugt solche in denen unter den Reaktionsbedingungen feste Stoffe als End- oder Zwischenprodukte gebildet werden. Ursache der Feststoffbildung ist eine lokale Übersättigung der feststoffbildenden Komponente gegenüber der Gleichgewichtslöslichkeit. Je schneller die Mischung desto höher auch die Übersättigung. Eine höhere Übersättigung führt zur Bildung von mehr Feststoffkeimen und gemeinhin zu kleineren Primärpartikeln. Handelt es sich dabei um ein Zwischenprodukt, so reagieren kleine Primärpartikel schneller weiter als große, da sie mehr Oberfläche aufweisen. Die Geschwindigkeit der anschließenden Umsetzung hängt somit maßgeblich von der Größe der gebildeten Partikeln ab. Für hohe Raum-Zeit-Aubeuten müssen daher im Mischorgan möglichst kleine Partikeln erzeugt werden. Weiterhin führt die Ausbildung größerer Partikel zur Gefahr der Bildung von Ablagerungen im Mischorgan. Zur Vermeidung von Feststoffablagerungen und zur Erzielung kurzer Mischzeiten sind daher kleine Grenzschichten anzustreben.

Dieses Prinzip ist sowohl für einphasige als auch mehrphasige, miteinander mischbare oder nicht-mischbare Medien anwendbar.

Mit Vorteil kann die erfindungsgemäße Vorrichtung in der Herstellung von Isocyanaten durch Umsetzung der korrespondierenden Amine mit Phosgen als Mischvorrichtung für die Vermischung von Amin und Phosgen eingesetzt werden. Dabei spielt es zunächst keine Rolle, ob die Reaktion in der Gas- oder in der Flüssigphase stattfindet, besonders vorteilhaft kann sie in der Gasphasenphosgenierung als Mischvorrichtung eingesetzt werden.

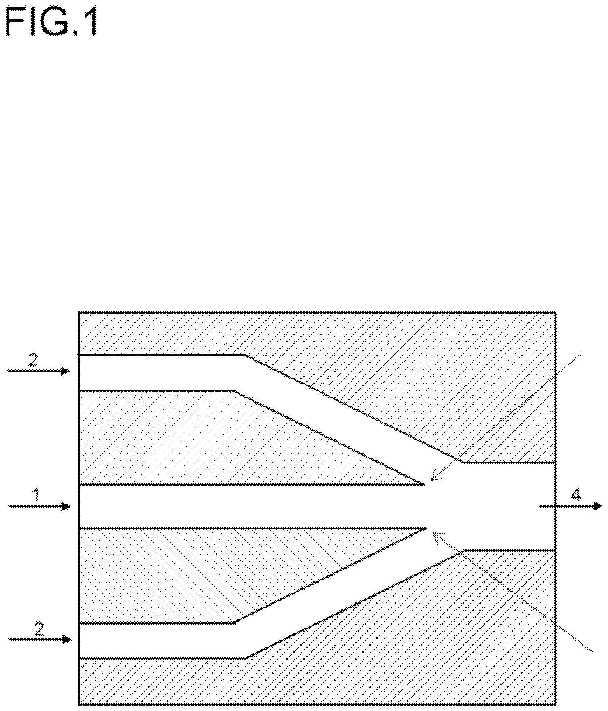

Bekannt ist es beispielsweise aus EP 289840 B1 oder aus EP 1275639 A1 die Vermischung von Amin und Phosgen in der Gasphasenphosgenierung mit Hilfe einer Kombination aus Düse und Ringspalt. Dieses Vermischungsprinzip ist exemplarisch gezeigt in Figur 1.

Es wurde nun gefunden, daß dadurch, daß man in der Mischeinrichtung zum Vermischen fluider Ströme von Amin, Phosgen zumindest zwischen einen Aminstrom und einen Phosgenstrom ein Inertmedium dosiert, die Abscheidung von verstopfend wirkenden Feststoffen im Bereich der Mischeinrichtung verringert bis unterdrückt werden kann.

Die größte Neigung zum Ausfall von Feststoffen ergibt sich in Mischeinrichtungen gemäß Figur 1 an der Stelle des ersten Kontaktes von Amin- und Phosgenstrom (siehe unbeschriftete Pfeile). Die Ursache hierfür können Ablösungen der Strömung (Rezirkulationsgebiete) am Kontaktpunkt oder aber das Überlagerungsgebiet der Grenzschichten der Zuläufe am Kontaktpunkt sein. In diesen Zonen ist die Verweilzeit erhöht. Phosgen und Amin reagieren zu Isocyanaten, die zu festen Folgeprodukten wie Harnstoffen, Diimiden oder Cyanuraten weiterreagieren können. Diese können sich im Bereich des Kontaktpunktes ablagern und zu einer Belagbildung im Mischorgan führen.

Durch die erfindungsgemäße Dosierung eines Intermediums zwischen die Ströme von Phosgen und Amin wird der Kontakt zwischen den beiden reagierenden Strömen in Wandnähe vermieden. Die Mischung erfolgt somit in einer Entfernung von der Wandung, so daß eine geringere Belagbildung die Folge ist.

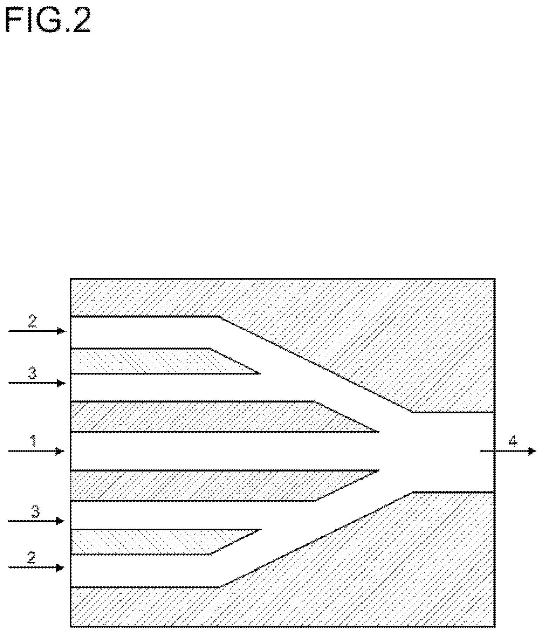

Eine Ausführungsform der vorliegenden Erfindung ist in Figur 2 dargestellt: Darin wird in einer Mischeinrichtung ein innerer Aminstrom 1 zwischen zwei Phosgenströme 2 dosiert. Erfindungsgemäß wird auf der Innenseite, d.h. auf der dem Aminstrom 1 zugewandten Seite der Phosgenstromkanäle 2 jeweils ein Inertmedium 3 so dosiert, dass bis zum Mündungspunkt des Kanals für das Amin keine vollständige Vermischung von Phosgen und Inertmedium eintritt. In diesem Fall ist die Phosgenkonzentration am Mündungspunkt des Amins herabgesetzt und idealerweise 0. In diesem Fall liegen am Mündungspunkt nur Amin und Inertmedium vor und die Bildung von festen Folgeprodukten kann vermieden werden.

Der Abstand der Inerteindosierung in den Phosgenkanal vom Mündungspunkt des A-minstroms sollte so gewählt werden, dass bis dahin keine vollständige Durchmischung von Inertmedium und Phosgen eingetreten ist. Bekanntermaßen ist die Mischlänge in turbulenter Strömung etwa das 50fache des hydraulischen Durchmessers der Kanalabmessung. Entsprechend sollte der Abstand der Inertdosierung vom Aminmündungspunkt kleiner als das 50fache des hydraulischen Durchmessers der Phosgenzuleitung am Mündungspunkt sein, bevorzugt kleiner als das 10 fache und besonders bevorzugt kleiner als das 5 fache sein.

Das Verhältnis der absoluten Geschwindigkeiten von Inertmedium und Phosgen am Kontaktpunkt beider Strömungen sollte zur Vermeidung einer zu schnellen Vermischung im Bereich von 2:1 bis 1:2, bevorzugt von 1,5:1 bis 1:1,5 und besonders bevorzugt 1,2:1 bis 1:1,2 betragen.

Bevorzugt beträgt die Geschwindigkeitsdifferenz der absoluten Geschwindigkeiten von Inertmedium und Phosgen weniger als 10 m/s, besonders bevorzugt weniger als 8 m/s und ganz besonders bevorzugt weniger als 5 m/s.

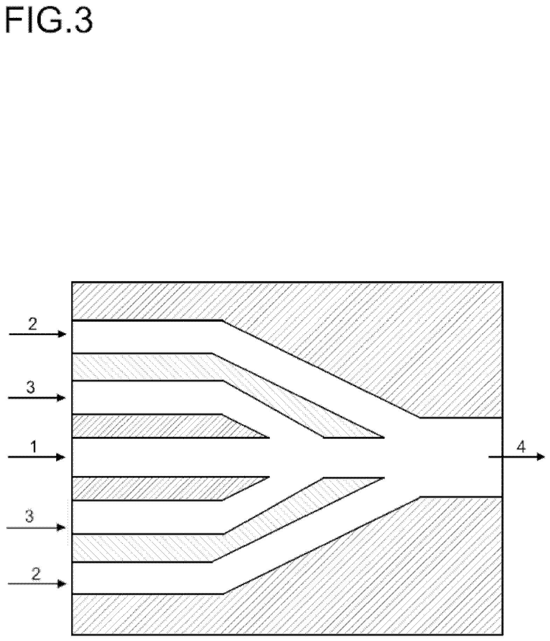

Eine weitere Ausführungsform der vorliegenden Erfindung ist in Figur 3 dargestellt: Darin wird in einer Mischeinrichtung ein innerer Aminstrom 1 zwischen zwei Phosgenströme 2 dosiert. Erfindungsgemäß wird auf der Außenseite, d.h. auf der dem Phosgenstrom 2 zugewandten Seite des Aminstromkanals 1 jeweils ein Inertmedium 3 so dosiert, dass bis zum Mündungspunkt des Aminkanals keine vollständige Vermischung von Amin und Inertmedium eintritt. In diesem Fall ist die Aminkonzentration am Mündungspunkt des Phosgenkanals 2 herabgesetzt und ist idealerweise 0. In diesem Fall liegen am Mündungspunkt nur Phosgen und Inertmedium vor und die Bildung von festen Folgeprodukten kann vermieden werden.

Der Abstand der Inerteindosierung in den Aminkanal 1 vom Mündungspunkt des Phosgenstroms 2 sollte so gewählt werden, dass bis dahin keine vollständige Durchmischung von Inertmedium und Amin eingetreten ist. Der Abstand der Inertdosierung vom Phosgenmündungspunkt sollte kleiner als das 50fache des hydraulischen Durchmessers der Aminzuleitung am Mündungspunkt sein, bevorzugt kleiner als das 10 fache und besonders bevorzugt kleiner als das 5 fache sein.

Das Verhältnis der absoluten Geschwindigkeiten von Inertmedium und Amin am Kontaktpunkt beider Strömungen sollte zur Vermeidung einer zu schnellen Vermischung im Bereich von 2:1 bis 1: 2, bevorzugt von 1,5:1 bis 1:1,5 und besonders bevorzugt 1,2:1 bis 1:1,2 betragen.

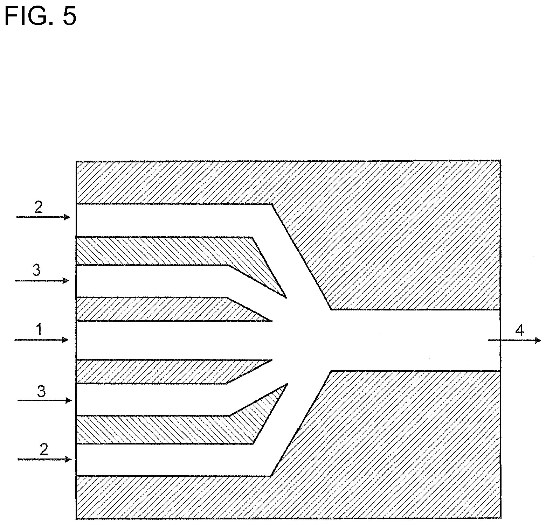

Eine weitere bevorzugte Ausführungsform der vorliegenden Erfindung ist in Figur 5 dargestellt: Darin wird in einer Mischeinrichtung ein innerer Aminstrom 1 zwischen zwei Phosgenströme 2 dosiert. Erfindungsgemäß wird zwischen dem Phosgenkanal 2 und dem Aminstromkanals 1 jeweils ein Inertmedium 3 dosiert. Dies bewirkt zunächst eine räumliche Trennung von Aminstrom 1 und Phosgenstrom 2, so daß am Mündungspunkt des Aminkanals 1 nur Amin und Inertmedium und an den Mündungspunkten der Phosgenkanäle 2 nur Phosgen und Inertmedium in Kontakt treten.

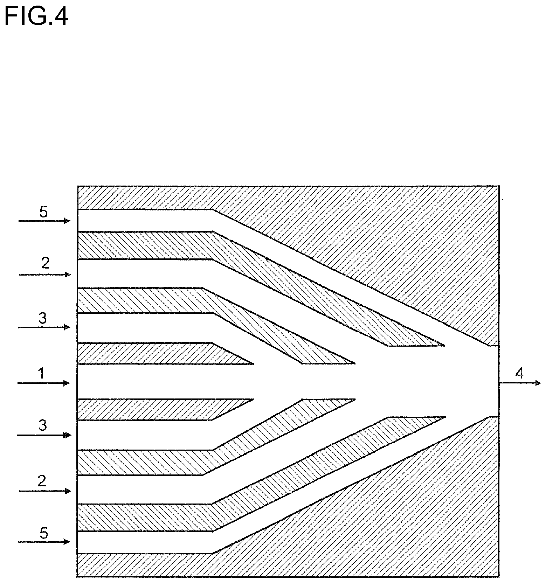

Zusätzlich zu dieser erfindungsgemäßen Trennung von Amin- und Phosgenstrom durch ein Inertmedium kann optional auch ein Inertstrom zwischen der Wandung und dem äußeren Phosgenstrom dosiert werden, wie es exemplarisch dargestellt ist in Figur 4: Dazu wird ein weiterer Strom 5 eines Inertmediums zwischen Phosgenstrom 2 und Wandung dosiert, während zwischen Aminstrom 1 und Phosgenstrom 2 ein Inertmedium 3 dosiert werden kann, wie in den Ausführungsformen der Figuren 1, 2 oder 5.

Dies bewirkt ein Fernhalten des Reaktionsgemisches von der Wandung, so daß gegebenenfalls intermediär gebildete Feststoffe sich nur vermindert oder nicht an der Wandung des Reaktionsraumes abscheiden können.

Dabei spielt der Abstand der Eindosierung des Inertmediums 5 in den Kanal des Reaktionsgemisches 4 von der Stelle der Zusammenführung mit dem Phosgenstrom 2 eine untergeordnete Rolle, solange das Reaktionsgemisch von der Wandung ferngehalten wird.

Eine derartige Dosierung eines Inertstroms zwischen Wandung und Reaktionsgemisch kann ein- oder mehrmals erfolgen, beispielsweise an ein bis vier Stellen des Reaktionsraums, bevorzugt an ein bis drei, besonders bevorzugt an ein bis zwei Stellen und ganz besonders bevorzugt an einer Stelle.

Eine derartige Dosierung eines Inertstroms zwischen Wandung und Reaktionsgemisch macht insbesondere für die Stellen des Reaktionsraums Sinn, an denen Amin und Phosgen lediglich bzgl. der Unterschußkomponenten teilumgesetzt sind, beispielsweise bei einem Teilumsatz bis zu 85%, bevorzugt bis zu 75%, besonders bevorzugt bis zu 50%, ganz besonders bevorzugt bis zu 30% und insbesondere bis zu 15%. In diesen Bereichen ist die Gefahr der Feststoffbildung besonders hoch, da im Verlauf der Reaktion freigesetzter Chlorwasserstoff mit freiem Amin reagieren kann und so Feststoffteilchen bilden kann, die auszufallen können. Außer diesen Aminhydrochloriden können Folgeprodukte aus Amin und Isocyanat oder Isocyanaten miteinander, beispielsweise Harnstoffe, Diimide oder Isocyanurate, zu Ablagerungen führen, deren Bildung durch die erfindungsgemäße Durchführung verringert werden kann.

Es is auch denkbar, wenn auch weniger bevorzugt, die vorliegende Erfindung lediglich mit einer Dosierung eines Inertmediums 5 zwischen Wandung und Reaktionsgemisch 4 ohne Dosierung eines Inertmediums 3 zwischen Phosgen- 2 und Aminstrom 1 auszuführen.

Bei der Mischeinrichtung kann es sich bevorzugt um statische Mischorgane, beispielsweise um eine Düsenmischeinrichtung, beispielsweise Koaxialmischdüsen, Y- oder T-Mischer, Strahlmischer oder Mischrohre handeln.

Bei einer Koaxialmischdüse wird in einem Mischrohr durch ein konzentrisches Rohr mit kleinem Duchmesser (Düse) mit hoher Geschwindigkeit die eine Komponente (bevorzugt das Amin) in die andere Komponente (dann bevorzugt Phosgen) geführt.

Bei den Reaktoren kann es sich beispielsweise um zylinderförmige Reaktionsräume ohne Einbauten und ohne bewegte Teile handeln.

Eine Ausführungsform einer Mischungs-/Reaktionseinheit ist beschrieben in EP 1275639 A1, dort besonders in den Absätzen [0013] bis [0021] und dem Beispiel zusammen mit Figur 1, was hiermit durch Bezugnahme Bestandteil der vorliegenden Offenbarung sei. Bevorzugt ist jedoch im Gegensatz zur dortigen Offenbarung die Dosierung des Amin durch das Innenrohr und von Phosgen als äußerem Strom.

Eine Ausführungsform einer Mischungs-/Reaktionseinheit ist beschrieben in EP 1275640 A1, dort besonders in den Absätzen [0010] bis [0018] und dem Beispiel zusammen mit Figur 1, was hiermit durch Bezugnahme Bestandteil der vorliegenden Offenbarung sei. Bevorzugt ist jedoch im Gegensatz zur dortigen Offenbarung die Dosierung des Amin durch das Innenrohr und von Phosgen als äußerem Strom.

Eine weitere Ausführungsform einer Mischungs-/Reaktionseinheit ist beschrieben in EP 1319655 A2, dort besonders in den Absätzen [0015] bis [0018] und dem Beispiel zusammen mit Figur 1, was hiermit durch Bezugnahme Bestandteil der vorliegenden Offenbarung sei.

Es kann sinnvoll sein, Strömungsvergleichmäßiger einzubauen, wie beschrieben in EP 1362847 A2, dort besonders in den Absätzen [0008] bis [0026] und dem Beispiel zusammen mit Figur 1, was hiermit durch Bezugnahme Bestandteil der vorliegenden Offenbarung sei.

Denkbar ist auch der Einsatz von mehreren parallel ausgerichteten Düsen, wie beschrieben in EP 1449826 A1, dort besonders in den Absätzen [0011] bis [0027] und Beispiel 2 zusammen mit den Figuren 1 bis 3, was hiermit durch Bezugnahme Bestandteil der vorliegenden Offenbarung sei.

Eine weitere Ausführungsform einer Mischungs-/Reaktionseinheit ist beschrieben in DE 10359627 A1, dort besonders in den Absätzen [0007] bis [0025] und Beispiel 1 zusammen mit der Figur, was hiermit durch Bezugnahme Bestandteil der vorliegenden Offenbarung sei.

Eine bevorzugte Ausführungsform für eine Mischdüse ist eine Schlitzmischdüse, wie sie beschrieben ist in der WO 2008/55895, dort besonders von Seite 3, Zeile 26 bis Seite 15, Zeile 31 und ein Reaktionsraum wie er dort beschrieben ist von Seite 15, Zeile 35 bis Seite 23, Zeile 31 zusammen mit den Figuren, was hiermit durch Bezugnahme Bestandteil der vorliegenden Offenbarung sei.

Bevorzugt handelt es sich um eine Schlitzmischdüse mit einer ungeradzahligen Anzahl von Schlitzen, beispielsweise 5, 7, 9 etc.

In derartigen Schlitzstapeln können die einzelnen Ströme beispielsweise in der folgenden Reihenfolge dosiert werden:
Phosgen - [ - Inertmedium - Amin - Inertmedium - Phosgen -]n mit n ≥ 1
oder
Inert - Phosgen - [ - Inert - Amin - Inert - Phosgen -]n - Inert mit n ≥ 1

Bevorzugt kann n dabei Werte von 1 oder 2 annehmen, besonders bevorzugt von 1.

Eine besonders bevorzugte Ausführungsform für eine Mischdüse ist eine Ringspaltmischdüse, wie sie beschrieben ist in der Internationalen Patentanmeldung WO 2007/028715, dort besonders von Seite 2, Zeile 23 bis Seite 11, Zeile 22 und ein Reaktionsraum wie er dort beschrieben ist von Seite 11, Zeile 26 bis Seite 21, Zeile 15 zusammen mit Figur 2, was hiermit durch Bezugnahme Bestandteil der vorliegenden Offenbarung sei.

Bevorzugt handelt es sich um eine Ringspaltmischdüse mit einer ungeradzahligen Anzahl von Ringspalten, beispielsweise 5, 7, 9 etc.

Die Reihenfolge der Dosierung kann analog wie bei den Schlitzstapeln angegeben erfolgen.

Wenn kein Inertmediumstrom 5 zwischen Phosgenstom 2 und Wandung dosiert wird, ist es vorteilhaft zur Vermeidung von Feststoffablagerung und Verstopfungen bei der erfindungsgemäßen Mischeinrichtung der phosgenhaltige Eduktstrom bevorzugt so zu führen, dass sämtliche Apparatewandungen nach Zusammenführung der Eduktströme von dem oder den phosgenhaltigen Eduktströmen überströmt werden und der oder die aminhaltigen Eduktströme vollständig von dem oder den phosgenhaltigen Eduktströmen solange umhüllt werden, bis eine vollständige Vermischung der Ströme oder ein weitgehend vollständiger Umsatz des Amins erfolgt ist.

Daher wird bevorzugt das Amin innen dosiert, so daß der Strom vollständig von allen Seiten von einem Phosgenstrom umgeben ist. Denkbar, wenn auch weniger bevorzugt, ist eine Dosierung, bei der Phosgen innen und Amin außen dosiert wird. In diesem Fall ist es bevorzugt zwischen der Wandung und dem Amin ein Inertmedium einzudosieren.

Die Amine, die in eine Gasphasenphosgenierung eingesetzt werden können, müssen bestimmte Erfordernisse erfüllen (siehe unten).

Dabei kann es sich um Monoamine, Diamine, Triamine oder höherwertige Amine handeln, bevorzugt um Diamine. Ensprechend ergeben sich die korrespondierenden Monoisocyanate, Diisocyanate, Triisocyanate oder höherwertigen Isocyanate, bevorzugt Diisocyanate.

Die Amine und Isocyanate können aliphatisch, cycloaliphatisch oder aromatisch sein, bevorzugt aliphatisch oder cycloaliphatisch und besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

(Cyclo)aliphatische Isocyanate stehen im Rahmen dieser Anmeldung kurz für cycloaliphatische und/oder aliphatische Isocyanate.

Beispiele für aromatische Diisocyanate sind bevorzugt solche mit 6-20 C-Atomen, beispielsweise monomeres 2,4'- oder 4,4'-Methylen-di(phenylisocyanat (MDI), 2,4- und/oder 2,6-Toluylendiisocyanat (TDI) und 1,5- oder 1,8-Naphtyldiisocyanat (NDI).

Diisocyanate sind bevorzugt (cyclo)aliphatische Diisocyanate, besonders bevorzugt (cyclo)aliphatische Diisocyanate mit 4 bis 20 C-Atomen.

Beispiele für übliche Diisocyanate sind aliphatische Diisocyanate wie 1,4-Tetramethylendiisocyanat, 1,5-Pentamethylendiisocyanat, Hexamethylendiisocyanat (1,6-Diisocyanatohexan), 1,8-Octamethylendiisocyanat, 1,10-Decamethylendiisocyanat, 1,12-Dodecamethylen-diisocyanat, 1,14-Tetradecamethylendiisocyanat, Derivate des Lysindiisocyanates, Tetramethylxylylendiisocyanat (TMXDI), Trimethylhexandiisocyanat oder Tetra-methylhexandiisocyanat, sowie 3 (bzw. 4), 8 (bzw. 9)-Bis(isocyanatomethyl)-tricyclo[5.2.1.0²⁶]decan-Isomerengemische, sowie cycloaliphatische Diisocyanate wie 1,4-, 1,3- oder 1,2-Diisocyanatocyclohexan, 4,4'- oder 2,4'-Di(isocyanatocyclo-hexyl)methan, 1-Isocyanato-3,3,5-trimethyl-5-(isocyanatomethyl)cyclohexan (Isophorondiisocyanat), 1,3- oder 1,4-Bis(isocyanatomethyl)cyclohexan, 2,4-, oder 2,6-Diisocyanato-1-methylcyclohexan.

Bevorzugt sind 1,5-Pentamethylendiisocyanat, 1,6-Diisocyanatohexan, 1-Isocyanato-3,3,5- trimethyl-5-(isocyanato-methyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5- trimethyl-5-(isocyanatomethyl)cyclohexan und 4,4'-Di(isocyanat-ocyclohexyl)methan.

Für das erfindungsgemäße Verfahren können solche Amine zur Reaktion zu den korrespondierenden Isocyanaten eingesetzt werden, bei denen das Amin, deren korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 mol%, besonders bevorzugt zu höchtens 1 mol% und ganz besonders bevorzugt zu höchstens 0,5 mol% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,5-Diaminopentan, 1,6-Diaminohexan, 1-Amino-3,3,5-trimethyl-5-aminomethylcyclohexan (IPDA) und 4,4'-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (mol/mol) Gemisch, Diaminobenzol, 2,6-Xylidin, Napthyldiamin (NDA) und 2,4'- oder 4,4'-Methylen(diphenylamin) (MDA) oder Isomerengemische davon. Bevorzugt sind unter diesen die Diamine, besonders bevorzugt ist 2,4- und/oder 2,6-TDA.

Bei der Gasphasenphosgenierung ist es definitionsgemäß anzustreben, daß die im Reaktionsverlauf auftretenden Verbindungen, also Edukte (Diamin und Phosgen), Zwischenprodukte (insbesondere die intermediär entstehenden Mono- und Dicarbamylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase z.B. an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für auftretende Aminhydrochloride, die sich aus freien Aminogruppen und Chlorwasserstoff (HCl) bilden, da die resultierenden Aminhydrochloride leicht ausfallen und nur schwer wieder verdampfbar sind.

Die Edukte, oder auch nur eines von ihnen, können zusätzlich zu der erfindungsgemäßen zusätzlichen Dosierung eines Inertmediums verdünnt mit mindestens einem Inertmedium in den Mischraum eindosiert werden.

Bei dem Inertmedium handelt es sich um ein Medium, das bei der Reaktionstemperatur gasförmig im Reaktionsraum vorliegt und nicht wesentlich mit den im Reaktionsverlauf auftretenden Verbindungen reagiert. Damit ist gemeint, daß weniger als 10 mol% des Inertmediums unter den Reaktionsbedingungen chemisch reagieren, bevorzugt weniger als 5 mol%, besonders bevorzugt weniger als 3 mol% und ganz besonders bevorzugt weniger als 1 mol%.

Das Inertmedium wird im Allgemeinen vor der Umsetzung mit Amin und/oder Phosgen vermischt, kann aber auch getrennt von den Eduktströmen zudosiert werden. Beispielsweise können Stickstoff, Edelgase, wie Helium oder Argon, oder Aromaten, wie Chlorbenzol, Chlortoluol, o-Dichlorbenzol, Toluol, Xylol, Chlornaphthalin, Decahydronaphthalin, Kohlenstoffdioxid oder Kohlenstoffmonoxid, verwendet werden. Bevorzugt wird Stickstoff und/oder Chlorbenzol als Inertmedium verwendet.

Im Allgemeinen wird das Inertmedium in einer Menge eingesetzt, so dass das Verhältnis der Gasvolumina von Inertmedium zu Amin bzw. zu Phosgen weniger als 1, bevorzugt weniger als 0,1 und besonders bevorzug weniger als 0,05 beträgt.

Die Ausgangsamine werden vor der Durchführung des erfindungsgemäßen Verfahrens verdampft und auf 200°C bis 600°C, vorzugsweise 300°C bis 500°C erhitzt und gegebenenfalls verdünnt mit einem Inertgas oder mit den Dämpfen eines inerten Lösungsmittels durch die Mischeinrichtung dem Reaktor zugeführt.

Das bei der Phosgenierung verwendete Phosgen wird vor Durchführung des erfindungsgemäßen Verfahrens gegebenenfalls verdünnt mit einem Inertgas oder mit den Dämpfen eines inerten Lösungsmittels ebenfalls auf eine Temperatur innerhalb des Bereichs von 200°C bis 600°C, vorzugsweise 300°C bis 500°C erhitzt.

Erfindungsgemäß wird Phosgen im Überschuss bezüglich Aminogruppen eingesetzt. Üblicherweise liegt ein molares Verhältnis von Phosgen zu Aminogruppen von 1,1 :1 bis 20 : 1, bevorzugt von 1,2 :1 bis 5 :1 vor.

Die Vermischung und Reaktion der beiden gasförmigen Edukte findet nach dem erfindungsgemäßen Verfahren nach der Einleitung der Eduktströme Diamin und Phosgen sowie Inertmedium über die Eintrittsflächen in dem Mischraum als Reaktionsraum statt.

Die Reaktion setzt in der Regel mit Kontakt der Edukte unmittelbar nach der Vermischung ein.

So findet im vorderen Teil des Reaktionsraums die Vermischung der Edukte, gegebenenfalls vermischt mit Inertmedium, statt (Mischraum).

Zur Durchführung der erfindungsgemäßen Umsetzung werden der vorerhitzte Strom enthaltend Amin oder Gemische von Aminen und der vorerhitzte Strom enthaltend Phosgen kontinuierlich in den Reaktor, bevorzugt einen Rohrreaktor geleitet.

Die Reaktoren bestehen im Allgemeinen aus Stahl, Glas, legiertem oder emaillierten Stahl und weisen eine Länge auf, die ausreichend ist, um unter den Verfahrensbedingungen eine vollständige Umsetzung des Diamins mit dem Phosgen zu ermöglichen.

Es kann sinnvoll sein, in den Eduktzuleitungen Strömungsvergleichmäßiger einzubauen, wie sie beispielsweise aus der EP 1362847 A bekannt sind. Bevorzugt wird zur Vergleichmäßigung der Geschwindigkeit der Eduktströme jedoch eine im Verhältnis zum Durchmesser der Zuleitung lange Vorlauflänge in der Eduktzuleitung, das 2 bis 40-fache des Zuleitungsdurchmessers, besonders bevorzugt das 4 bis 30-fache, ganz besonders bevorzugt das 5 bis 20-fache.

Eine wie in Patent EP 1275640A1 beschriebene Verengung des Strömungsquerschnittes nach der Zusammenführung der Eduktströme zur Vermeidung von Rückstömungen ist möglich, bevorzugt kann jedoch darauf verzichtet werden.

Damit ein Aminstrom dem Erfindungsgedanken folgend nach Beginn der Vermischung keinen Kontakt mit den Apparatewandungen hat, sondern von phosgenhaltigen Eduktströmen umhüllt ist, wird der Aminstrom zwischen Phosgenströme dosiert. Zusätzlich kann die Apparatewandung durch einen zusätzlichen Strom 5 eines Inertmediums geschützt werden.

Die Strömungsquerschnitte des oder der phosgenhaltigen Eduktströme werden so gestaltet, dass das charakteristische Mischungslängenmaß wieder möglichst klein wird. Da das Edukt Phosgen im stöchiometrischen Überschuss zugeführt wird und außerdem die Phosgengeschwindigkeit bevorzugt kleiner als die Amingeschwindigkeit ist, muss eine größere Querschnittsfläche als für den aminhaltigen Strom gewählt werden, woraus sich auch größere charakteristische Abmaße ergeben. Die Mischungsweglänge wird kleiner als 200 mm, bevorzugt kleiner als 100 mm, besonders bevorzugt kleiner als 50 mm, ganz besonders bevorzugt kleiner als 25 mm und insbesondere kleiner als 10 mm gewählt. Die Mischungsweglänge ist dabei als die Distanz definiert, die Fluidelemente zweier oder mehrerer Eduktströme senkrecht zur Fliessrichtung der Eduktströme maximal zurücklegen müssen bis eine molekulare Vermischung der Eduktströme erfolgt ist.

Das Verhältnis der Gesamtfläche der Aminströme zur Gesamtfläche der Phosgenströme ist größer als 0,00002, bevorzugt größer als 0,0002, besonders bevorzugt größer als 0,002 und ganz besonders bevorzugt größer als 0,02.

Das Verhältnis der Gesamtfläche der Aminströme zur Gesamtfläche der Phosgenströme ist kleiner als 5, bevorzugt kleiner als 1, besonders bevorzugt kleiner als 0,5 und ganz besonders bevorzugt kleiner als 0,2.

Das Flächenverhältnis zweier phosgenführender Flächen, die durch einen aminführenden Schlitz getrennt sind, beträgt 0,1 bis 10, bevorzugt 0,2 bis 5, besonders bevorzugt 0,4 bis 2,5, ganz besonders 0,8 bis 1,25, insbesondere 0,9 bis 1,1 und speziell 1.

Das Verhältnis der Fläche eines Inertmediumstromes zur Gesamtfläche des benachbarten Amin- und Phosgenstromes ist größer als 0,0002, bevorzugt größer als 0,002 und besonders bevorzugt größer als 0,02 und beträgt bis zu 5, bevorzugt bis zu 2 und besonders bevorzugt bis zu 1.

Da die Intensität und Schnelligkeit der Vermischung der amin- und phosgenhaltigen Eduktströme wesentlich vom sich in der Mischzone einstellenden Schergradienten abhängen, muss die Mischzone so gestaltet werden, dass der Schergradient besonders groß ist.

Dazu sollte einerseits die Differenzgeschwindigkeit zwischen den amin- und phosgenhaltigen Eduktströmen besonders hoch gewählt werden und zum anderen die charakteristischen Längenabmaße möglichst klein gewählt werden, da der Schergradient proportional zum Quotienten aus Geschwindigkeitsdifferenz und charakteristischem Längenmaß ist.

Die Geschwindigkeitsdifferenz zwischen dem Strom des Inertmediums und dem benachbarten Strom sollte jedoch nicht so groß sein, dass ein ausgedehntes Rezirkulationsgebiet durch den eintretenden Strahl ausgebildet wird.

Da die Geschwindigkeitsdifferenz zwischen den amin- und phosgenhaltigen E-duktsströmen hoch sein soll, müssen entweder die phosgenhaltigen oder die aminhaltigen Eduktströme eine größere Geschwindigkeit aufweisen. Da die Aminzuführungen zur Mischzone empfindlicher gegenüber der Bildung von Ablagerungen und Verstopfungen sind und eine Rückströmung in der Aminzuführung auf jeden Fall zu vermeiden ist, wird die Strömungsgeschwindigkeit des aminhaltigen Eduktstromes bevorzugt größer gewählt als die Geschwindigkeit des phosgenhaltigen Eduktstromes.
Je höher die Geschwindigkeit der aminhaltigen Eduktströme ist um so höher kann bei gleichem Schergefälle auch die Geschwindigkeit der phosgenhaltigen Eduktströme gewählt werden. Eine höhere Phosgengeschwindigkeit bewirkt kleinere Strömungsquerschnitte der Phosgenzuführung und damit kleinere Mischungsweglängen und somit ein schnelleres Mischen.

Um eine möglichst hohe Amingeschwindigkeit zu erreichen, wird daher angestrebt im Aminstrom am Punkt der Zusammenführung mit dem Phosgenstrom eine lokale Machzahl von größer als 0,6 einzustellen.

Die Machzahl bedeutet dabei das Verhältnis zwischen lokaler Strömungsgeschwindigkeit und lokaler Schallgeschwindigkeit. In einer besonderen Ausführungsform des Verfahrens wird die Zuführung des aminhaltigen Eduktströme so gewählt, dass am Austritt der Aminströme in die Mischzone gerade eine Machzahl von 1 vorliegt.

Im Fall einer sogenannten angepassten Aminzuführung entspricht der Druck des Aminstromes an diesem Punkt gerade dem Druck, des phosgenhaltigen Eduktstroms am Punkt der Zusammenführung. Im Falle einer nichtangepassten Aminzuführung ist der Druck des Aminströmes beim Austritt aus der Aminzuführung größer als der Druck des phosgenhaltigen Stromes bei der Zusammenführung. In diesem Fall kommt es dann zu einer weiteren Expansion des aminhaltigen Stromes, der mit einer Druckabsenkung bis auf den Druck des phosgenhaltigen Stromes verbunden ist.
Ob eine Düse angepasst oder nicht angepasst betrieben wird, hängt vom Vordruck des aminhaltigen- und des phosgenhaltigen Stromes vor der Mischdüse ab.
In einer weiteren besonderen Ausführungsform ist die Aminzuführung so gestaltet, dass bereits in der Zuführungen Machzahlen von größer 1 erreicht werden. Dies kann z.B. dadurch erreicht werden, dass die Zuführung des aminhaltigen Ströme in Form einer oder mehrer Lavaldüsen gestaltet wird, die sich dadurch auszeichnen, dass sich der Strömungsquerschnitt zunächst verengt bis eine Machzahl von eins erreicht ist und sich, dann wieder erweitert, was zu einer weiteren Expansion und Beschleunigung der Strömung führt. Um eine Überschallströmung (Machzahl größer als 1) zu erreichen, muss das Verhältnis des Aminkesseldruckes zum Mischzonendruck größere als das sogenannte kritische Druckverhältnis sein. Je höher das Druckverhältnis ist und je höher die Kesseltemperatur des Aminstromes ist, umso höher ist die maximal erreichbare Geschwindigkeit.
Da das Edukt Amin bei zu hohen Temperaturen oft thermisch geschädigt wird, dürfen allerdings keine zu hohen Temperaturen eingestellt werden. Auch kann der Aminvordruck auf Grund des Amindampfdruckes nicht beliebig gesteigert werden.
Bevorzugt wird die Aminzuführung daher so gestaltet, dass sich im aminhaltigen Eduktstrom direkt an der Zusammenführung mit dem phosgenhaltigen Strom oder im Falle einer nicht angepassten Düse kurz dahinter Machzahlen von 0,6 bis 4, besonders bevorzugt 0,7 bis 3, ganz besonders bevorzugt 0,8 bis 2,5 und insbesondere 0,9 bis 2,0 einstellen.
Die angegebenen Machzahlen kann der Fachmann bei bekannter Kesseltemperatur und bekannten Stoffdaten einfach in Strömungsgeschwindigkeiten umrechnen. Ebenso kann der Fachmann in Abhängigkeit von der angegebenen Machzahl und den Stoffdaten den erforderlichen Vordruck berechnen.

Die hohe Eintrittsgeschwindigkeit des Aminstromes in die Mischzone dient, wie oben dargestellt, der Erzielung einer möglichst großen Geschwindigkeitsdifferenz zwischen amin- und phosgenhaltigen Eduktströmen. Ferner wird durch die hohe Strömungsgeschwindigkeit der Systemdruck und damit auch die Eduktkonzentrationen sowie die Temperatur lokal reduziert, was zu einer Verringerung der Reaktionsgeschwindigkeiten und damit zu einer Vereinfachung der Mischaufgabe führt.
Um möglichst kleine Mischungsweglängen zu erreichen, muss angestrebt werden, die Strömungsgeschwindigkeit des phosgenhaltigen Eduktstromes ebenfalls möglichst hoch zu wählen, ohne jedoch die Differenzgeschwindigkeit zwischen amin- und phosgenhaltigem Eduktstrom zu sehr zu reduzieren. Dazu wird die Querschnittsfläche des Phosgenstromes so gewählt, dass sich eine Machzahl von 0,2 bis 2,0, bevorzugt 0,3 bis 1,5, besonders bevorzugt 0,4 bis 1,0, ganz besonders bevorzugt von 0,5 bis 1,0 und insbesondere 0,7 bis 1,0 ergibt.
Die Strömungsquerschnitte der aminhaltigen Eduktströme werden in der erfindungsgemäßen Mischeinheit so gestaltet, dass einerseits eine hohe Betriebstabilität gewährleistet ist und anders möglichst kleine Mischungsweglängen eingehalten werden. Daher werden für die Zuführung des aminhaltigen Eduktsstromes charakteristische Längenmaße von 0,5 bis 50 mm, bevorzugt 0,75 bis 25 mm, besonders bevorzugt 1 mm bis 10 mm und ganz besonders bevorzugt 1 mm bis 5 mm gewählt. Als charakteristisches Längenmaß ist dabei der kleinste Längenmaßstab des Strömungsquerschnitts gemeint, d.h. z.B. im Fall eines Spaltes die Spaltbreite oder im Fall einer kreisförmigen Öffnung der Öffnungsdurchmesser.

Bevorzugt werden die einzelnen Edukte in der Mischeinrichtung mit einer Strömungsgeschwindigkeit von 20 bis 400 Meter/Sekunde in den Reaktor geführt, bevorzugt von 25 bis 300 Meter/Sekunde, besonders bevorzugt 30 bis 250, ganz besonders bevorzugt 50 bis 200, insbesondere mehr als 150 bis 200 und speziell 160 bis 180 Meter/Sekunde.

In einer möglichen Ausführungsform der Erfindung kann es sinnvoll sein, die Phosgenströme, insbesondere den äußeren Phosgenstrom mit einer höheren Strömungsgeschwindigkeit in den Mischraum einzuführen, als den Aminstrom, den sie umhüllen, besonders bevorzugt mit mindestens 10 m/s mehr, ganz besonders bevorzugt mindestens 20 m/s mehr und insbesondere mindestens 50 m/s mehr.

Es kann aber auch möglich und sinnvoll sein, den äußeren Phosgenstrom mit einer höheren Strömungsgeschwindigkeit in den Mischraum einzuführen, als den Aminstrom, und den inneren Phosgenstrom mit einer niedrigeren Strömungsgeschwindigkeit. Dies stellt eine weitere mögliche Ausführungsform der vorliegenden Erfindung dar.

In einer bevorzugten Ausführungsform der Erfindung ist es sinnvoll, die Phosgenströme, insbesondere den äußere Phosgenstrom mit einer niedrigeren Strömungsgeschwindigkeit in den Mischraum einzuführen, als den Aminstrom, den sie umhüllen, besonders bevorzugt mit mindestens 50 m/s weniger, ganz besonders bevorzugt mindestens 60 m/s weniger, ganz besonders bevorzugt 80 m/s weniger und im speziellen mindestens 100 m/s weniger.

In einer bevorzugten Ausführungsform der vorliegenden Erfindung sind bei einer Vielzahl von Phosgenströme diese mit genau einer Phosgenzuleitung druckverlustarm ohne zusätzliche Regeleinrichtungen verbunden, so daß die Geschwindigkeit, mit der das Phosgen strömt, etwa gleich ist.

Desgleichen gilt, daß bei einer Vielzahl von Aminströmen diese bevorzugt mit genau einer Aminzuleitung druckverlustarm ohne zusätzliche Regeleinrichtungen verbunden sind, so daß die Geschwindigkeit, mit der das Amin strömt, etwa gleich ist.

Es ist aber auch möglich, die Phosgen- und/oder Aminströme der Schlitze mit jeweils einer getrennt geregelten Zuleitung zu verbinden, so daß die Geschwindigkeiten pro Zuleitung einzeln und unabhängig voneinander einstellbar sind.

Die Edukte treten mit einem Geschwindigeitsvektor in den Mischraum. Dabei lässt sich der Geschwindigkeitsvektor in eine axiale, radiale und tangentiale Richtungskomponente unterteilen. Unter der axialen Richtung wird die Richtungskomponente des Geschwindigkeitsvektors parallel zur Längsachse des Mischraums verstanden. Unter der radialen Richtung wird die Richtungskomponente des Geschwindigkeitsvektors von aussen auf die Längsachse zu verstanden, also einen rechten Winkel mit der Längsachse einschließend. Unter tangentialer Richtung wird die Richtungskomponente des Geschwindigkeitsvektors parallel zur Berandung des Mischraums verstanden, also eine ringförmige Umlaufbewegung.

Zur Vermischung der Eduktströme läßt sich eine Verbesserung der sich einstellenden Vermischung durch den Einbau von eine Tangentialgeschwindigkeit erzeugenden Elementen erzielen, beispielsweise in die Zuleitung der Teilströme der Überschußkomponenten in den Mischraum. Ein geeignetes tangentialgeschwindigkeitserzeugendes Element wäre beispielsweise ein in die Zuleitung eingelassenes spiralförmig verdrilltes Band (Wendel), runde oder eckige Leitbleche (Leitschaufeln) oder dergleichen. Die Wirkung der tangentialgeschwindigkeitserzeugenden Einbauten ist es, in der Strömung der Düse die Scherung zwischen Strömungsschichten unterschiedlicher Zusammensetzung zu erhöhen.

Es kann eine weitere Ausführungsform darstellen, auch den Strom des Inertmediums mit einem Drall zu versehen. Dies ist jedoch weniger bevorzugt.

Zur Erzeugung einer Tangentialgeschwindigkeit ist auch ein tangentialer Eintritt der Zuleitung eines oder mehrerer Eduktströme möglich oder bei einem radialen Zustrom eines oder mehrerer Eduktströme ein Schaufelkranz.

Weiterhin kann es sinnvoll sein, die Phosgen- und Aminströme mit gegenläufiger Tangentialgeschwindigkeit in den Mischraum einzuleiten, beispielsweise indem die Phosgenströme mit einer Tangentialgeschwindigkeit entlang der Längsachse des Reaktors blickend im Uhrzeigersinn, und der zwischenliegende Aminstrom mit einer Tangentialgeschwindigkeit gegen den Uhrzeigersinn in den Mischraum eindosiert werden.

Der Winkel, den der Summenvektor aus den Vektoren der Tangentialgeschwindigkeit und aus dem Vektor der Axialgeschwindigkeit der so eindosierten Ströme mit der Längsachse des Reaktors einschließt, kann von 5 bis 85°, bevorzugt 17 bis 73°, besonders bevorzugt 30 bis 60° für die einen Ströme, beispielsweise die Phosgenströme, und von -5 bis -85°, bevorzugt -17 bis -73°, besonders bevorzugt -30 bis -60° für die anderen Ströme, beispielsweise den Aminstrom betragen.

Weiterhin ist es sinnvoll, die Strömungen mit unterschiedlichen Radialgeschwindigkeiten in den Mischraum einzudosieren. Dabei stellt sich ein Winkel zwischen dem Summenvektor aus dem Radialgeschwindigkeitsvektor und aus dem Axialgeschwindigkeitsvektor mit der Längsachse ein. Dieser Winkel entspricht in der Regel dem Winkel des zugehörigen Dosierkanals mit der Längsachse des Mischraums. Dabei bedeutet eine negativer Winkel eine Dosierung von innen nach außen, ein positiver Winkel eine Dosierung von außen nach innen, ein Winkel von 0° bedeutet eine zur Längsachse des Mischraums parallele Strömung und ein Winkel von 90° eine zur Längsachse des Mischraums senkrechte Strömung.

Der äußere Phosgenstrom kann durch die Mischeinrichtung unter einem radialen Winkel von 0 bis 90°, bevorzugt 5 bis 90°, besonders bevorzugt 7 bis 65°, ganz besonders bevorzugt 15 bis 35° und insbesondere 18 bis 30° in den Mischraum eindosiert werden.

Es stellt eine bevorzugte Ausführungsform der vorliegenden Erfindung dar, den Phosgenstrom in einem Winkel von etwa 90°, bevorzugt genau 90° zur Längsachse des Mischraums und den Aminstrom parallel Längsachse des Mischraums zu dosieren. Dabei wird der Aminstrom von einem Inertmedium eingehüllt, das bevorzugt über einen Ringspalt um den Aminstrom dosiert wird.

Der Aminstrom kann durch die Mischeinrichtung unter einem radialen Winkel von -50° bis +50°, bevorzugt -25 bis 25°, besonders bevorzugt -10 bis 10° und ganz besonders bevorzugt -3 bis +3° in den Mischraum eindosiert werden.

Der innere Phosgenstrom kann durch die Mischeinrichtung unter einem radialen Winkel von 0 bis -85°, bevorzugt -5 bis -85°, besonders bevorzugt -7 bis -65°, ganz besonders bevorzugt -15 bis -35° und insbesondere -18 bis -30° in den Mischraum eindosiert werden.

Vorteilhaft ist es, wenn äußerer Phosgenstrom und Aminstrom relativ zueinander einen radialen Winkel von 1 bis 60°, bevorzugt 7 bis 50, besonders bevorzugt 15 bis 45° und besonders bevorzugt 18 bis 35° einschließen.

Weiterhin vorteilhaft ist es, wenn Aminstrom und innerer Phosgenstrom relativ zueinander einen radialen Winkel von 1 bis 60°, bevorzugt 10 bis 50°, besonders bevorzugt 15 bis 45° und besonders bevorzugt 18 bis 35° einschließen.

Um einen möglichst vollständigen Umsatz des Amins zum jeweiligen Wertprodukt zu erreichen, wird mit den oben dargestellten Maßnahmen eine Mischzeit des phosgenhaltigen mit dem aminhaltigen Eduktstromes von kleiner 10 ms, bevorzugt kleiner 5 ms, besonders bevorzugt kleiner 2 ms, ganz besonders bevorzugt kleiner 1 ms und insbesondere kleiner 0,5 ms erreicht. Als Mischzeit wird dabei die Zeit definiert, die Fluidelemente, die aus der Aminzuführung austreten maximal benötigen, bis sich in ihnen ein Phosgen/Aminverhältnis von größer oder gleich 4 einstellt. Die Zeit zählt dabei jeweils ab dem Austritt eines Fluidelements aus der Aminzuführung.

### Reaktionsraum

Der Reaktionsraum umfaßt im vorderen Bereich den Mischraum, in dem überwiegend die Vermischung des gasförmigen Gemisches von Phosgen, Amin, gegebenenfalls vermischt mit Inertmedium, und getrennt durch Ströme von Inertmedium stattfindet, was in der Regel begleitet ist vom Einsetzen der Reaktion. Im hinteren Teil des Reaktionsraums findet dann im Wesentlichen nur noch die Reaktion statt und höchstens untergeordnet die Vermischung.

Zu Unterscheidungszwecken kann als Mischraum der Bereich des Reaktionsraumes bezeichnet werden, in dem die Vermischung der Edukte zu einem Grad von 99% stattfindet. In einer bevorzugten Ausführungsform der vorliegenden Erfindung ist der Umsatz im Mischraum, d.h. der Verbrauch des eingesetzten Amins, weniger als 15%. Dabei wird der Vermischungsgrad als Verhältnis der Differenz des lokal gemittelten Mischungsbruch und des Anfangsmischungsbruchs vor Vermischung zur Differenz des mittleren endgültigen Mischungsbruchs nach Vermischung und des Anfangsmischungsbruchs vor Vermischung angegeben. Zum Konzept des Mischungsbruches siehe z.B. J. Warnatz, U. Maas, R.W. Dibble: Verbrennung, Springer Verlag, Berlin Heidelberg New York, 1997, 2. Auflage, S. 134.

Der Effekt der erfindungsgemäßen Zudosierung des Inertmediums zwischen einen Amin- und einen Phosgenstrom bewirkt lediglich eine verminderte Vermischung dieser beiden Komponenten im Bereich der Mischeinrichtung. In einer bestimmten Entfernung von der Mischeinrichtung findet dann eine normale Vermischung von Phosgen und Amin statt.

Unter Reaktor wird die technische Vorrichtung verstanden, die den Reaktionsraum enthält. Hierbei kann es sich um alle üblichen, aus dem Stand der Technik bekannten Reaktionsräume handeln, die zur nicht katalytischen, einphasigen Gasreaktion, bevorzugt zur kontinuierlichen nicht katalytischen, einphasigen Gasreaktion, geeignet sind und die den geforderten moderaten Drücken standhalten. Geeignete Materialien für den Kontakt mit dem Reaktionsgemisch sind z.B. Metalle, wie Stahl, Tantal, Nickel, Nickellegierungen, Silber oder Kupfer, Glas, Keramik, Emaille oder homogene oder heterogene Gemische daraus. Bevorzugt werden Stahlreaktoren verwendet. Die Wände des Reaktors können hydraulisch glatt oder profiliert sein. Als Profile eignen sich beispielsweise Ritzen oder Wellen.

Es kann von Vorteil sein, wenn das eingesetzte Material, bevorzugt das für die Mischeinrichtung und/oder den Reaktor und besonders bevorzugt das für den Reaktor eingesetzte Material eine geringe Rauhigkeit aufweist, wie beschrieben in der unveröffentlichten Europäischen Patentanmeldung mit dem Aktenzeichen 06125811.7 und dem Einreichedatum 11.12.2006, auf die hiermit im Rahmen der vorliegenden Offenbarung vollständig Bezug genommen sei.

Es können im Allgemeinen die aus dem Stand der Technik bekannten Reaktorbautypen verwendet werden. Beispiele für Reaktoren sind bekannt aus EP-B1 289840, Sp. 3, Z. 49 - Sp. 4, Z. 25, EP-B1 593334, WO 2004/026813, S. 3, Z. 24 - S. 6, Z. 10, WO 03/045900, S. 3, Z. 34 - S. 6, Z. 15, EP-A1 1275639, Sp. 4, Z. 17 - Sp. 5, Z. 17 und EP-B1 570799, Sp. 2, Z. 1 - Sp. 3, Z. 42, auf die jeweils im Umfang dieser Offenbarung ausdrücklich Bezug genommen sei.

Bevorzugt verwendet werden Rohrreaktoren.

Ebenfalls ist es möglich, im Wesentlichen quaderförmige Reaktionsräume, bevorzugt Plattenreaktoren bzw. Plattenreaktionsräume zu verwenden. Ein besonders bevorzugter Plattenreaktor weist ein Verhältnis von Breite zu Höhe von mindestens 2 : 1, bevorzugt mindestens 3 : 1, besonders bevorzugt mindestens 5 : 1 und insbesondere mindestens 10 : 1 auf. Die obere Grenze des Verhältnisses von Breite zu Höhe hängt von der gewünschten Kapazität des Reaktionsraums ab und ist prinzipiell nicht begrenzt. Als technisch sinnvoll haben sich Reaktionsräume mit einem Verhältnis von Breite zu Höhe bis zu 5000 : 1, bevorzugt bis zu 1000 : 1 erwiesen.

Die Umsetzung von Phosgen mit Amin im Reaktionsraum erfolgt bei Absolutdrücken von mehr als 0,1 bar bis weniger als 20 bar, bevorzugt zwischen 0,5 bar und 15 bar und besonders bevorzugt zwischen 0,7 und 10 bar. Im Fall der Umsetzung von (cyclo)aliphatischen Aminen beträgt der Absolutdruck ganz besonders bevorzugt zwischen 0,7 bar und 5 bar, insbesondere von 0,8 bis 3 bar und speziell 1 bis 2 bar.

Im Allgemeinen ist der Druck in den Zuleitungen zur Mischvorrichtung höher, als der vorstehend angegebene Druck im Reaktor. Je nach Wahl der Mischvorrichtung fällt an dieser Druck ab. Bevorzugt ist der Druck in den Zuleitungen um 20 bis 2000 mbar, besonders bevorzugt von 30 bis 1000 mbar höher als im Reaktionsraum.

In einer möglichen Ausführungsform besteht der Reaktor aus einem Bündel an Reaktoren. In einer möglichen Ausführungsform muss es sich bei der Mischeinheit nicht um eine eigenständige Vorrichtung handeln, vielmehr kann es vorteilhaft sein, die Mischeinheit in den Reaktor zu integrieren. Ein Beispiel einer integrierten Einheit aus Mischeinheit und Reaktor stellt ein Rohrreaktor mit angeflanschten Düsen dar.

Gemäß dem erfindungsgemäßen Verfahren erfolgt die Umsetzung von Phosgen mit Amin in der Gasphase. Unter Umsetzung in der Gasphase ist zu verstehen, dass die Umwandlung der Eduktströme und Zwischenprodukte zu den Produkten im gasförmigen Zustand miteinander reagieren und im Verlauf der Reaktion während des Durchgangs durch den Reaktionsraum zu mindestens 95%, bevorzugt zu mindestens 98%, besonders bevorzugt zu mindestens 99%, ganz besonders bevorzugt zu mindestens 99,5%, insbesondere zu mindestens 99,8 und speziell zu mindestens 99,9% in der Gasphase bleiben.

Zwischenprodukte sind dabei beispielsweise die aus den Diaminen gebildeten Monoamino-monocarbamoylchloride, Dicarbamoylchloride, Monoamino-monoisocyanate und Monoisocyanato-monocarbamoylchloride sowie die Hydrochloride der Aminoverbindungen.

Bei dem erfindungsgemäßen Verfahren wird die Temperatur im Reaktionsraum so gewählt, dass sie oberhalb der Siedetemperatur des eingesetzten Diamins, bezogen auf die im Reaktionsraum herrschenden Druckverhältnisse, liegt. Je nach eingesetztem Amin und eingestelltem Druck ergibt sich üblicherweise eine vorteilhafte Temperatur im Reaktionsraum von mehr als 200 °C, bevorzugt mehr als 260 °C und besonders bevorzugt mehr als 300 °C. In der Regel beträgt die Temperatur bis zu 600 °C, bevorzugt bis zu 570 °C.

Die mittlere Kontaktzeit des Umsetzungsgemisches im erfindungsgemäßen Verfahren beträgt im Allgemeinen zwischen 0,001 Sekunden und weniger als 5 Sekunden, bevorzugt mehr als 0,01 Sekunden bis weniger als 3 Sekunden, besonders bevorzugt mehr als 0,015 Sekunden bis weniger als 2 Sekunden. Im Fall der Umsetzung von (cyclo)aliphatischen Aminen kann die mittlere Kontaktzeit ganz besonders bevorzugt von 0,015 bis 1,5 Sekunden, insbesondere von 0,015 bis 0,5 Sekunden, speziell von 0,020 bis 0,1 Sekunden und oft von 0,025 bis 0,05 Sekunden betragen.

Unter mittlerer Kontaktzeit wird die Zeitspanne vom Beginn der Vermischung der Edukte bis zum Verlassen des Reaktionsraumes in die Aufarbeitungsstufe verstanden. In einer bevorzugten Ausführungsform ist die Strömung im Reaktor des erfindungsgemäßen Verfahrens durch eine Bodenstein-Zahl von mehr als 10, bevorzugt mehr als 100 und besonders bevorzugt von mehr als 500 charakterisiert.

In einer bevorzugten Ausführungsform werden die Abmessungen des Reaktionsraums und die Strömungsgeschwindigkeiten so gewählt, dass eine turbulente Strömung, d.h. eine Strömung mit einer Reynolds-Zahl von mindestens 2300, bevorzugt mindestens 2700, für das Reaktionsgemisch vorliegt, wobei die Reynolds-Zahl mit dem hydraulischen Durchmesser des Reaktionsraumes gebildet wird.

Bevorzugt durchläuft das gasförmige Reaktionsgemisch den Reaktionsraum mit einer Strömungsgeschwindigkeit von 10 bis 300 Meter/Sekunde, bevorzugt von 25 bis 250 Meter/Sekunde, besonders bevorzugt 40 bis 230, ganz besonders bevorzugt 50 bis 200, insbesondere mehr als 150 bis 190 und speziell 160 bis 180 Meter/Sekunde.

Durch die turbulente Strömung werden enge Verweilzeitverteilungen mit geringer Standardabweichung von meist nicht mehr als 6% wie in EP 570799 beschrieben und eine gute Vermischung erreicht. Maßnahmen, wie beispielsweise die in EP-A-593 334 beschriebene Verengung, die zudem verstopfungsanfällig ist, sind nicht notwendig.

Es kann sinnvoll sein, in den Reaktor Strömungsvergleichmäßiger einzubauen, wie sie beispielsweise aus der EP 1362847 A bekannt sind.

Das Reaktionsvolumen kann über seine Außenfläche temperiert werden. Um Produktionsanlagen mit hoher Anlagenkapazität zu bauen, können mehrere Reaktorrohre parallel geschalten werden. Die Umsetzung kann aber auch bevorzugt adiabat erfolgen. Das bedeutet, dass über die Außenfläche des Reaktionsvolumens nicht mit technischen Maßnahmen Heiz- oder Kühlenergieströme fließen.

In einer bevorzugten Ausführungsform werden die Umsetzungsbedingungen so gewählt, dass das Reaktionsgas am Austritt aus dem Reaktionsraum eine Phosgenkonzentration von mehr als 25 mol/m³, bevorzugt von 30 bis 50 mol/m³, aufweist. Weiterhin liegt am Austritt aus dem Reaktionsraum im Allgemeinen eine Inertmediumskonzentration von mehr als 25 mol/m³, bevorzugt von 30 bis 100 mol/m³ vor.

Der Reaktionsraum kann einen gleichbleibenden Durchmesser aufweisen oder im Verlauf der Durchströmung eine Serie von Verengungen oder Erweiterungen aufweisen. Dies ist beispielsweise beschrieben in WO 2007/028715, Seite 14, Zeile 29 bis Seite 20, Zeile 42, was hiermit ausdrücklich Bestandteil der vorliegenden Offenbarung sei.

Die Ausführung des Reaktionsraums spielt jedoch erfindungsgemäß auf die Vermischung der Komponenten keine Rolle.

Das durchströmte Volumen des Reaktors kann mit statischen Mischern ausgefüllt sein, beispielsweise Packungen, Formkörpern, Geweben, Loch- oder Schlitzblechen, bevorzugt ist das Volumen jedoch möglichst frei von Einbauten.

Denkbar ist auch der Einbau von Leitblechen in den Reaktionsraum. Ein geeignetes, turbulenzerzeugendes Element wäre beispielsweise ein eingelassenes spiralförmig verdrilltes Band, runde oder eckige Schrägplatten oder dergleichen.

Um auch bei großen Amin- und Phosgenmengenströmen, wie sie bei der Isocyanatproduktion im großtechnischen Maßstab üblich sind, kleine Mischungsweglängen und damit kurze Mischzeiten einzuhalten, bietet sich eine Parallelschaltung vieler kleiner Mischdüsen mit anschließender Misch- und Reaktionszone an, wobei die parallelgeschalteten Einheiten durch Wandungen von einander getrennt sind. Der Vorteil dieser Verfahrensvariante liegt in einem günstigeren Längen- zu Durchmesserverhältnis der Misch- und Reaktionszonen. Je größer dieses Verhältnis ist, desto günstiger (enger) ist die Verweilzeitverteilung der Strömung. Somit kann bei gleicher Verweilzeit und Strömungsgeschwindigkeit durch viele parallelgeschaltete Einheiten, dass Längen- zu Durchmesserverhältnis erhöht werden und damit auch die Verweilzeitverteilung eingeengt werden. Um den apparativen Aufwand gering zuhalten, münden die einzelnen Reaktionszonen in eine gemeinsame Nachreaktionszone, in der dann der Restumsatz des Amins erfolgt.

### Quench

Nach der Reaktion wird das gasförmige Umsetzungsgemisch bevorzugt bei Temperaturen größer 130 °C mit einem Lösungsmittel gewaschen (Quench). Als Lösungsmittel sind bevorzugt Kohlenwasserstoffe, die gegebenenfalls mit Halogenatomen substituiert sind, geeignet, wie beispielsweise Hexan, Benzol, Nitrobenzol, Anisol, Chlorbenzol, Chlortoluol, o-Dichlorbenzol, Trichlorbenzol, Diethylisophthalat (DEIP), Tetrahydrofuran (THF), Dimethylformamid (DMF), Xylol, Chlornaphthalin, Decahydronaphthalin und Toluol. Als Lösungsmittel wird besonders bevorzugt Monochlorbenzol eingesetzt. Als Lösungsmittel kann auch das Isocyanat eingesetzt werden. Bei der Wäsche wird das Isocyanat selektiv in die Waschlösung übergeführt. Anschließend werden das verbleibende Gas und die erhaltene Waschlösung bevorzugt mittels Rektifikation in Isocyanat, Lösungsmittel, Phosgen und Chlorwasserstoff aufgetrennt.

Nachdem das Reaktionsgemisch im Reaktionsraum umgesetzt wurde, führt man es in die Aufarbeitungsvorrichtung mit Quench. Bevorzugt handelt es sich hier um einen sogenannten Waschturm, wobei aus dem gasförmigen Gemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Bevorzugt wird dabei die Temperatur des inerten Lösungsmittels oberhalb der Lösungstemperatur des zum Amin gehörigen Carbamylchlorids im gewählten Quenchmedium gehalten. Besonders bevorzugt wird dabei die Temperatur des inerten Lösungsmittels oberhalb der Schmelztemperatur des zum Amin gehörigen Carbamylchlorids gehalten

Im Allgemeinen ist der Druck in der Aufarbeitungsvorrichtung niedriger als im Reaktionsraum. Bevorzugt ist der Druck um 50 bis 500 mbar, besonders bevorzugt 80 bis 150 mbar, niedriger als im Reaktionsraum.

Die Wäsche kann beispielsweise in einem Rührbehälter oder in anderen herkömmlichen Apparaturen, z.B. in einer Kolonne oder Mixer-Settler-Apparatur, durchgeführt werden.

Verfahrenstechnisch können für eine Wäsche im erfindungsgemäßen Verfahren alle an sich bekannten Extraktions- und Waschverfahren und -apparate eingesetzt werden, z.B. solche, die in Ullmann's Encyclopedia of Industrial Chemistry, 6th ed, 1999 Electronic Release, Kapitel: Liquid - Liquid Extraction - Apparatus, beschrieben sind. Beispielsweise können dies ein- oder mehrstufige, bevorzugt einstufige Extraktionen, sowie solche in Gleich- oder Gegenstromfahrweise, bevorzugt Gegenstromfahrweise sein.

Der Quench kann beispielsweise ausgeführt sein wie beschrieben in EP 1403248 A1, dort besonders in den Absätzen [0006] bis [0019] und dem Beispiel zusammen mit den Figuren 1 bis 2, was hiermit durch Bezugnahme Bestandteil der vorliegenden Offenbarung sei.

Der Quench kann beispielsweise ausgeführt sein wie beschrieben in der WO 2008/55899, dort besonders von Seite 3, Zeile 30 bis Seite 11, Zeile 37 zusammen mit Beispiel 1 und den Figuren, was hiermit durch Bezugnahme Bestandteil der vorliegenden Offenbarung sei.

Der Quench kann beispielsweise ausgeführt sein wie beschrieben in der WO 2008/55904, dort besonders von Seite 3, Zeile 26 bis Seite 16, Zeile 36 zusammen mit Beispiel 1 und den Figuren, was hiermit durch Bezugnahme Bestandteil der vorliegenden Offenbarung sei.

Der Quench kann bevorzugt ausgeführt sein wie beschrieben in WO 2005/123665, dort besonders von Seite 3, Zeile 10 bis Seite 8, Zeile 2 und dem Beispiel, was hiermit durch Bezugnahme Bestandteil der vorliegenden Offenbarung sei.

In dieser Quenchzone wird das Reaktionsgemisch, das im Wesentlichen aus den Isocyanaten, Phosgen und Chlorwasserstoff besteht, intensiv mit der eingedüsten Flüssigkeit vermischt. Die Vermischung erfolgt derart, dass die Temperatur des Reaktionsgemisches ausgehend von 200 bis 570°C auf 100 bis 200 °C, bevorzugt auf 140 bis 180 °C abgesenkt wird und das im Reaktionsgemisch enthaltene Isocyanat durch Kondensation vollständig oder teilweise in die eingedüsten Flüssigkeitströpfchen übergeht, während das Phosgen und der Chlorwasserstoff im Wesentlichen vollständig in der Gasphase verbleiben.

Der Anteil des im gasförmigen Reaktionsgemisch enthaltenen Isocyanats, das in der Quenchzone in die Flüssigphase übergeht, beträgt dabei vorzugsweise 20 bis 100 Gew.-%, besonders bevorzugt 50 bis 99,5 Gew.-% und insbesondere 70 bis 99 Gew.-%, bezogen auf das im Reaktionsgemisch enthaltene Isocyanat.

Das Reaktionsgemisch durchströmt die Quenchzone vorzugsweise von oben nach unten. Unterhalb der Quenchzone ist ein Sammelbehälter angeordnet, in dem die Flüssigphase abgeschieden, gesammelt und, über einen Auslass aus dem Reaktionsraum entfernt und anschließend aufgearbeitet wird. Die verbleibende Gasphase wird über einen zweiten Auslass aus dem Reaktionsraum entfernt und ebenfalls aufgearbeitet.

Der Quench kann beispielsweise erfolgen, wie in der EP 1403248 A1 beschrieben, oder wie in der internationalen Anmeldung WO 2005/123665 beschrieben.

Die Flüssigkeitströpfchen werden dazu mittels Ein- oder Zweistoffzerstäuberdüsen, vorzugsweise Einstoffzerstäuberdüsen, erzeugt und erzeugen je nach Ausführungsform einen Sprühkegelwinkel von 10 bis 140°, bevorzugt von 10 bis 120°, besonders bevorzugt von 10° bis 100°.

Die Flüssigkeit, die über die Zerstäuberdüsen eingedüst wird, muss eine gute Löslichkeit für Isocyanate aufweisen. Vorzugsweise werden organische Lösungsmittel eingesetzt. Insbesondere eingesetzt werden aromatische Lösungsmittel, die mit Halogenatomen substituiert sein können.

In einer besonderen Ausführungsform des Verfahrens handelt es sich bei der eingedüsten Flüssigkeit um ein Gemisch aus Isocyanaten, ein Gemisch aus Isocyanaten und Lösungsmittel oder um Isocyanat, wobei die jeweils verwendete Quenchflüssigkeit Anteile an Leichtsieder, wie HCl und Phosgen, aufweisen kann. Vorzugsweise wird dabei das Isocyanat eingesetzt, das bei dem jeweiligen Verfahren hergestellt wird. Da durch die Temperatursenkung in der Quenchzone die Reaktion zum Stillstand kommt, können Nebenreaktionen mit den eingedüsten Isocyanaten ausgeschlossen werden. Der Vorteil dieser Ausführungsform liegt insbesondere darin, dass auf eine Abtrennung des Lösungsmittels verzichtet werden kann.

In einer alternativen bevorzugten Ausführungsform handelt es sich bei dem Inertmedium, das zusammen mit mindestens einem der Edukte eingesetzt wird, und bei dem Lösungsmittel, das im Quench eingesetzt wird, um die gleiche Verbindung, ganz besonders bevorzugt wird in diesem Fall Monochlorbenzol verwendet.

Geringe Mengen von Nebenprodukten, die im Isocyanat verbleiben, können mittels zusätzlicher Rektifikation, durch Strippen mit einem Inertgas oder auch Kristallisation, bevorzugt durch Rektifikation vom erwünschten Isocyanat getrennt werden.

In der anschließenden optionalen Reinigungsstufe wird das Isocyanat, bevorzugt durch Destillation, vom Lösungsmittel abgetrennt. Ebenfalls kann hier noch die Abtrennung von restlichen Verunreinigungen, umfassend Chlorwasserstoff, Inertmedium und/oder Phosgen, erfolgen, wie beispielsweise beschrieben in DE-A1 10260092.

### Beispiel

In einer Miniplant-Anlage zur Gasphasenphosgenierung von 1,6-Hexamethylendiamin zu 1,6-Hexamethylendiisocyanat wurden 1,77 kg/h eines Gemisches aus 99,2 Gew-% 1,6-Hexamethylendiamin und 0,8 Gew% Stickstoff auf 365°C erwärmt und und in einer Koaxialmischdüse mit 15 kg/h Phosgen mit einer Temperatur von 365°C vermischt. Die Mündung des zentralen Aminzulaufs hatte einen Durchmesser von 1,0 mm und das Phosgen wurde über einen Ringspalt von 3 mm in ein Mischrohr des Durchmessers 5,2 mm zugeführt.

Beim Betrieb der Anlage zeigte sich bereits nach wenigen Minuten ein Druckverlustanstieg, der auf Ablagerungen im Düsenbereich zurückzuführen war und letztlich zur Abstellung der Anlage führte.

Erfindungsgemäß wurde daraufhin ein Ringspalt von 1 mm zur Dosierung von ca. 0,35 kg/h Stickstoff als Inertmedium zwischen der zentralen Aminöffnung und dem Phosgenspalt installiert. Die Anlage konnte daraufhin über mehrere Stunden ohne nennenswerten Druckverlustanstieg betrieben werden. Nach Ausbau und Zerlegen der Düse konnten keine Ablagerungen im Bereich der Mischzone gefunden werden.

### Figuren

Figur 1: Vermischung von Amin und Phosgen in der Gasphasenphosgenierung mit Hilfe einer Kombination aus Düse und Ringspalt
Figur 2: Ausführungsform der vorliegenden Erfindung
Figur 3: Ausführungsform der vorliegenden Erfindung
Figur 4: Ausführungsform der vorliegenden Erfindung
Figur 5: Ausführungsform der vorliegenden Erfindung

### Liste der Bezugszeichen in den Figuren:

- 1: Aminstrom
- 2: Phosgenstrom
- 3: Inertmedium
- 4: Reaktionsgemisch
- 5: Inertmedium

## Patentansprüche

1. Verfahren zur Herstellung von Isocyanaten durch Umsetzung der korrespondierenden Amine mit Phosgen in Gegenwart von mindestens einem Inertmedium in der Gasphase, durch Inkontaktbringen fluider Ströme von Amin, Phosgen sowie Inertmedium in mindestens einer Mischeinrichtung und anschließende Reaktion von Amin und Phosgen miteinander, **dadurch gekennzeichnet, daß** man in der Mischeinrichtung zwischen jeden vorhandenen Aminstrom und jeden vorhandenen Phosgenstrom ein Inertmedium dosiert, daß die Dosierung des Inertmediums in einen Phosgenstrom in einem Abstand vom Aminstrom stattfindet und diese Dosierung so erfolgt, daß bis zum Mündungspunkt des Kanals für das Amin keine vollständige Vermischung von Phosgen und Inertmedium eintritt.

2. Verfahren gemäß Anspruch 1, **dadurch gekennzeichnet, daß** man zwischen einen fluiden Aminstrom **1** und zwei fluide Phosgenströme **2** jeweils einen Strom **3** eines Inertmediums dosiert.

3. Verfahren gemäß Anspruch 2, **dadurch gekennzeichnet, daß** man auf der dem Aminstrom **1** zugewandten Seite der Phosgenstromkanäle **2** jeweils ein Inertmedium **3** in den Phosgenstrom **2** dosiert.

4. Verfahren gemäß Anspruch 2, **dadurch gekennzeichnet, daß** man auf den dem Phosgenstrom **2** zugewandten Seiten des Aminstromkanals **1** ein Inertmedium **3** in den Aminstrom **1** dosiert.

5. Verfahren gemäß einem der vorstehenden Ansprüche, **dadurch gekennzeichnet, daß** man zusätzlich einen weiteren Strom **5** eines Inertmediums zwischen Phosgenstrom **2** und Wandung dosiert.

6. Verfahren gemäß einem der vorstehenden Ansprüche, **dadurch gekennzeichnet, daß** es sich bei der Mischeinrichtung um eine Ringspaltmischdüse handelt.

7. Verfahren gemäß einem der Ansprüche 1 bis 5, **dadurch gekennzeichnet, daß** es sich bei der Mischeinrichtung um eine Schlitzdüse handelt.

8. Verfahren gemäß einem der Ansprüche 1 bis 5, **dadurch gekennzeichnet, daß** in der Mischeinrichtung ein Phosgenstrom in einem Winkel von etwa 90° zur Längsachse des Mischraums und ein Aminstrom parallel Längsachse des Mischraums dosiert wird, wobei der Aminstrom von einem Inertmedium eingehüllt wird.

9. Verfahren gemäß einem der Ansprüche 1 bis 5, **dadurch gekennzeichnet, daß** die Mischung in einer Mischvorrichtung erfolgt, ausgewählt aus der Gruppe bestehend aus Koaxialmischdüsen, Y-Mischer, T-Mischer, Strahlmischer und Mischrohre.

10. Verfahren gemäß einem der vorstehenden Ansprüche, **dadurch gekennzeichnet, daß** das Inertmedium ausgewählt ist aus der Gruppe bestehend aus Stickstoff, Helium, Argon, Chlorbenzol, Chlortoluol, o-Dichlorbenzol, Toluol, Xylol, Chlornaphthalin, Decahydronaphthalin, Kohlenstoffdioxid und Kohlenstoffmonoxid.

11. Verwendung von Dreistoffmischdüsen zur Dosierung fluider Ströme von Inertmedium zwischen fluide Ströme von jedem vorhandenen Aminstrom und jedem vorhandenen Phosgenstrom in der Gasphasenphosgenierung.

12. Verwendung von Dreistoffmischdüsen zur Dosierung fluider Ströme von Inertmedium zwischen fluide Ströme von jedem vorhandenen Aminstrom und jedem vorhandenen Phosgenstrom in der Flüssigphasenphosgenierung.

## Claims

1. A process for preparing isocyanates by reacting the corresponding amines with phosgene in the presence of at least one inert medium in the gas phase, by contacting fluid streams of amine, phosgene and inert medium in at least one mixing device and then reacting amine and phosgene with one another, wherein an inert medium is metered in between each amine stream present and each phosgene stream present in the mixing device, the metering of the inert medium into a phosgene stream takes place at a distance from the amine stream and this metered addition is effected such that complete mixing of phosgene and inert medium does not occur up to the opening point of the channel for the amine.

2. The process according to claim 1, wherein in each case one stream **3** of an inert medium is metered in between one fluid amine stream **1** and two fluid phosgene streams **2.**

3. The process according to claim 2, wherein in each case one inert medium **3** is metered into the phosgene stream **2** on the side of the phosgene stream channels **2** facing the amine stream **1.**

4. The process according to claim 2, wherein an inert medium **3** is metered into the amine stream **1** on the sides of the amine flow channel **1** facing the phosgene stream **2.**

5. The process according to any of the preceding claims, wherein a further stream **5** of an inert medium is additionally metered in between phosgene stream **2** and wall.

6. The process according to any of the preceding claims, wherein the mixing device is an annular gap mixing nozzle.

7. The process according to any of claims 1 to 5, wherein the mixing device is a slot nozzle.

8. The process according to any of claims 1 to 5, wherein a phosgene stream is metered in in the mixing device at an angle of about 90° relative to the longitudinal axis of the mixing chamber, and an amine stream parallel longitudinal axis of the mixing chamber, the amine stream being enclosed by an inert medium.

9. The process according to any of claims 1 to 5, wherein the mixing is effected in a mixing apparatus selected from the group consisting of coaxial mixing nozzles, Y mixers, T mixers, jet mixers and mixing tubes.

10. The process according to any of the preceding claims, wherein the inert medium is selected from the group consisting of nitrogen, helium, argon, chlorobenzene, chlorotoluene, o-dichlorobenzene, toluene, xylene, chloronaphthalene, decahydronaphthalene, carbon dioxide and carbon monoxide.

11. The use of three-substance mixing nozzles for metered addition of fluid streams of inert medium between fluid streams of each amine stream present and each phosgene stream present in gas phase phosgenation.

12. The use of three-substance mixing nozzles for metered addition of fluid streams of inert medium between fluid streams of each amine stream present and each phosgene stream present in liquid phase phosgenation.

## Revendications

1. Procédé pour la préparation de diisocyanates par transformation des amines correspondantes avec du phosgène en présence d'au moins un milieu inerte en phase gazeuse, par mise en contact de flux fluides d'amine, de phosgène ainsi que de milieu inerte dans au moins un dispositif de mélange et par réaction consécutive de l'amine et du phosgène l'un avec l'autre, **caractérisé en ce qu'**on dose un milieu inerte dans le dispositif de mélange, entre chaque flux d'amine présent et chaque flux de phosgène présent, **en ce que** le dosage du milieu inerte dans un flux de phosgène a lieu à une certaine distance du flux d'amine et ce dosage est effectué de manière telle que jusqu'à l'endroit où débouche le canal pour l'amine, il ne se produit pas de mélange complet du phosgène et du milieu inerte.

2. Procédé selon la revendication 1, **caractérisé en ce qu'**on dose à chaque fois un flux 3 d'un milieu inerte entre un flux 1 fluide d'amine et deux flux 2 fluides de phosgène.

3. Procédé selon la revendication 2, **caractérisé en ce qu'**on dose à chaque fois un milieu inerte 3 dans le flux 2 de phosgène du côté orienté vers le flux 1 d'amine des canaux 2 d'écoulement de phosgène.

4. Procédé selon la revendication 2, **caractérisé en ce qu'**on dose un milieu inerte 3 dans le flux 1 d'amine au niveau des côtés orientés vers le flux 2 de phosgène du canal 1 d'écoulement d'amine.

5. Procédé selon l'une quelconque des revendications précédentes, **caractérisé en ce qu'**on dose en outre un autre flux 5 d'un milieu inerte entre le flux 2 de phosgène et la paroi.

6. Procédé selon l'une quelconque des revendications précédentes, **caractérisé en ce qu'**il s'agit, pour le dispositif de mélange, d'un pulvérisateur de mélange à fente annulaire.

7. Procédé selon l'une quelconque des revendications 1 à 5, **caractérisé en ce qu'**il s'agit, pour le dispositif de mélange, d'un pulvérisateur à fente.

8. Procédé selon l'une quelconque des revendications 1 à 5, **caractérisé en ce qu'**un flux de phosgène est dosé dans le dispositif de mélange sous un angle d'environ 90° par rapport à l'axe longitudinal de la chambre de mélange et un flux d'amine est dosé parallèlement l'axe longitudinal de la chambre de mélange, le flux d'amine étant enveloppé d'un milieu inerte.

9. Procédé selon l'une quelconque des revendications 1 à 5, **caractérisé en ce que** le mélange est effectué dans un dispositif de mélange choisi dans le groupe constitué par les pulvérisateurs de mélange coaxiaux, les mélangeurs en Y, les mélangeurs en T, les mélangeurs à jet et les tubes mélangeurs.

10. Procédé selon l'une quelconque des revendications précédentes, **caractérisé en ce que** le milieu inerte est choisi dans le groupe constitué par l'azote, l'hélium, l'argon, le chlorobenzène, le chlorotoluène, l'o-dichlorobenzène, le toluène, le xylène, le chloronaphtalène, le décahydronaphtalène, le dioxyde de carbone et le monoxyde de carbone.

11. Utilisation de pulvérisateurs de mélange à trois substances pour le dosage de flux fluides de milieu inerte entre des flux fluides de chaque flux d'amine présent et de chaque flux de phosgène présent dans la phosgénation en phase gazeuse.

12. Utilisation de pulvérisateurs de mélange à trois substances pour le dosage de flux fluides de milieu inerte entre des flux fluides de chaque flux d'amine présent et de chaque flux de phosgène présent dans la phosgénation en phase liquide.
